# EUROPEAN PATENT SPECIFICATION

(11) **EP 2 400 904 B1**
(45) Date of publication and mention of the grant of the patent: **07.05.2014**
(21) Application number: 10706454.5
(22) Date of filing: 25.02.2010
(51) Int. Cl.: A61B 17/70

(54) **VERTEBRAL ROD SYSTEM AND METHODS OF USE**
WIRBELSTANGENSYSTEM UND VERWENDUNGSVERFAHREN DAFÜR
SYSTÈME DE TIGE VERTÉBRALE ET PROCÉDÉS D'UTILISATION

(30) Priority: 27.02.2009 US 394362
(43) Date of publication of application: 04.01.2012
(73) Proprietor: Warsaw Orthopedic, Inc., Warsaw, IN 46581 (US)
(72) Inventor: TRIEU, Hai H., Cordova Tennessee 38016 (US)
(74) Representative: Viering, Jentschura & Partner Patent- und Rechtsanwälte
(86) International application number: PCT/US2010/025409
(87) International publication number: WO 2010/099316

(56) References cited:
- WO-A1-2008/082085
- US-A1- 2007 191 832
- US-A1- 2008 234 736

## Description

### BACKGROUND

Spinal disorders such as degenerative disc disease, disc herniation, osteoporosis, spondylolisthesis, stenosis, scoliosis and other curvature abnormalities, kyphosis, tumor, and fracture may result from factors including trauma, disease and degenerative conditions caused by injury and aging. Spinal disorders typically result in symptoms including pain, nerve damage, and partial or complete loss of mobility.

Non-surgical treatments, such as medication, rehabilitation and exercise can be effective, however, may fail to relieve the symptoms associated with these disorders. Surgical treatments of these spinal disorders include discectomy, laminectomy, fusion and implantable prosthetics. As part of these surgical treatments, connecting elements such as vertebral rods are often used to provide stability to a treated region. During surgical treatment, one or more rods may be attached to the exterior of two or more vertebral members.

Rods redirect stresses away from a damaged or defective region while healing takes place to restore proper alignment and generally support the vertebral members. In some applications, rods are attached to the vertebral members without the use of implants or spinal fusion. Flexible connecting elements are also known that permit limited spinal motion of a spinal motion segment. Such flexible connecting elements can provide dynamic spinal support. While prior connecting elements have attempted to provide effective spinal tabilization, there remains a need for connecting elements that provide a dynamic stabilizing resistance to forces and permit motion of a spinal column segment(s) in flexion and extension while effectively stabilizing the spinal column segment(s) and the structural integrity of the connecting element.

Therefore, it would be desirable to provide a dynamic vertebral rod system, having flexion and extension capability, which provides stability while reducing stress on spinal elements. Desirably, the vertebral rod system includes a resistance member that provides resistance to motion and stress on the vertebral rod. It would be most desirable if the vertebral rod system includes a tension element to resist motion and stress. It would be highly desirable if characteristics such as rod stiffness, range of motion and fatigue strength of the system are adjustable.

US 2007/0191832 A1 discloses a vertebral rod comprising a first elongated section defining a first thichness, a second elongated section defining a second thickness, an intermediate section disposed between the first and second sections and defining a third thickness, the third thickness having a dimension being less than a dimension of at least one of the first and second thicknesses, the intermediate section having an inner surface that defines an open end, and a resistance member having an exterior surface configured for engaging at least a portion of the inner surface.

Further vertebral rods are disclosed, for example, in WO 2008/082085 A1 and US 2008/0234736 A1.

### SUMMARY OF THE INVENTION

The invention provides a vertebral rod according to claim 1. Further embodiments are described in the dependent claims. Any method or procedure described herein does as such not form a part of the invention.

Accordingly, a dynamic vertebral rod system is provided, having flexion and extension capability, which provides stability while reducing stress on spinal elements. Desirably, the vertebral rod system includes a resistance member that provides resistance to motion and stress on the vertebral rod. It is contemplated that the vertebral rod system includes a tension element to resist motion and stress. It is further contemplated that characteristics such as rod stiffness, range of motion and fatigue strength of the vertebral rod system are adjustable. It is envisioned that the disclosed system may be employed as a posterior, anterior and/or lateral dynamic stabilization device. The components of the vertebral rod system are easily manufactured and assembled.

In one embodiment, the vertebral rod system includes a dynamic vertebral rod with flexion and extension capabilities and methods of use. The vertebral rod includes upper and lower sections that are separated by a relatively flexible intermediate section. The intermediate section includes one or more members, and may have a variety of configurations to provide greater flexibility than the upper and lower sections. An elastic resistance member may be positioned within the intermediate section. The intermediate section and/or the elastic resistance member provide for variable resistance during movement of the upper and lower sections.

In an alternate embodiment, the resistance increases as the upper and lower sections move from a first orientation to a second orientation, which may include a load or forces applied to the sections in flexion and/or extension. In another embodiment, the extent of movement of the upper and lower sections is limited. The rod can be made of various materials including metals, polymers, ceramics and/or their composites. The elastic resistance member can be made of various polymers including silicone, polyurethane, silicone-polyurethane, polymeric rubbers and hydrogels.

In another embodiment, the rod is formed of a thermoplastic resin such as polyetheretherketone (PEEK), PEK, carbon-PEEK composite, PEEK-BaSO₄ and has a curved and flexible intermediate section encasing a polyurethane bumper. The upper and lower sections of the PEEK rod may be oval or round in cross-section. The intermediate section has a C-shape with the upper and lower sections connected near an open end of the intermediate section such that the overall length of the rod increases in spinal flexion and decreases in spinal extension.

In another alternate embodiment, a tension band such as a cable, tether, sleeve and/or jacket may be used to connect the upper and lower sections to limit motion and stress to the intermediate section in spinal extension. Rod stiffness, range of motion and fatigue strength can be adjustable. Other variations in rod configurations and materials are also contemplated to achieve similar flexion-extension capabilities.

In one embodiment, the vertebral rod can be manufactured via injection molding using a PEEK material and injection molding the bumper using a polyurethane material. Assembly includes inserting the bumper into the rod.

In an alternate embodiment, the intermediate section may be modified to modulate or change its stiffness or compliance, to correspondingly alter similar characteristics of the rod. Such modifications can include modifying the thickness of the cross-section of the rod; modifying the shape or profile of a particular cross-section of the rod; defining particular patterns in a surface of the rod such as a wave (or peak/valley), grooves, bumps, ribs, ridges; applying thermal treatment(s) to the rod; increasing resistance reinforcement of the rod with a tension band, tether or a cable. It is contemplated that the bumper can be of various sizes or shapes, such as cylindrical, spherical, rectangular or other regular or irregular shapes. It is further contemplated that the bumper can be fabricated from materials including polymers, elastomers, metals or ceramics or combinations thereof. Alternatively, the bumper can be solid, porous and may be designed to include patterns to modify modulus, stiffness or compliance. Various structure for securing the bumper with the rod are also contemplated, such as nonlocking screws or other features.

Alternatively, the vertebral rod system can include a non-locking multi-axial screw and a rod having sections with end stops, which allow the vertebral rod to slide within the screw under flexion-extension motion to cooperate with the flexion/bending of the rod. The non-locking screw provides an anti-disengagement or non-slip out of the rod from the screw. Other anti-disengagement configurations, such as a longer end-cap, an end bumper, or stoppers to limit sliding or prevent the rod from slipping off the screw are also envisioned.

The rod has an angled orientation and may have multiple/variable rod lengths to provide topping-off or trimming during surgery. It is envisioned that parameters of the rod system such as rod stiffness can be altered by modifying rod and/or bumper parameters such as material, material modulus, thickness, profile, component design and porosity. Accordingly, the vertebral rod system may be modular and/or adjustable by providing variable rod stiffness and/or bumper stiffness. It is contemplated that the rod has a static shear strength capable of resisting forces of at least 100 newtons (N), preferably at least 200N, and most preferably at least 400N, applied to the rod, with a rod deflection of at least 2 millimeters (mm), preferably at least 5mm, and at least 10mm without failure.

In one particular embodiment, in accordance with the principles of the present disclosure, a vertebral rod is provided. The vertebral rod includes a first elongated section defining a first thickness. A second elongated section defines a second thickness. An intermediate section is disposed between the first section and the second section and defines a third thickness. The third thickness has a dimension being less than a dimension of at least one of the first thickness and the second thickness. The intermediate section has an inner surface that defines an open end. A resistance member has an exterior surface configured for engaging at least a portion of the inner surface.

It is envisioned that at least one of the first and second thicknesses are a diameter. The intermediate section may define a width relative to each of the first and second thicknesses such that the dimension of the third thickness is less than or equal to a dimension of the width. The intermediate section may define a cross-sectional area based on the dimension of the third thickness and the first section can define a cross-sectional area based on the dimension of the first thickness and the second section can defines a cross-sectional area based on the dimension of the second thickness such that the cross sectional area of the intermediate section is greater than or equal to 10% of the cross sectional area of at least one of the first section and the second section.

The dimension of the width can be greater than or equal to at least one of the dimension of the first thickness and the second thickness. The open end can define an opening height between the first and second sections such that a dimension of the height is greater than or equal to 25% of the dimension of the at least one of the first and second thickness. The inner surface can define a mid region disposed an offset distance from longitudinal axes of the first and second sections adjacent the open end such that the offset distance is greater than or equal to 50% of the dimension of the at least one of the first and second thickness.

The inner surface can define a mid line disposed an offset distance from longitudinal axes of the first and second sections adjacent the open end such that the offset distance is greater than or equal to 50% of the dimension of at least one of the first and second thickness.

In an alternate embodiment, the vertebral rod includes a flexible intermediate section disposed between the first section and the second section. The flexible intermediate section has an arcuate inner surface that defines an elliptically shaped cavity and includes a locking part. An oblong bumper is mounted with the locking part and disposed within the cavity for engagement with the inner surface in a configuration that provides increasing resistance to movement of the first and second sections from a first orientation.

In another alternate embodiment, the vertebral rod includes an intermediate section having an inner surface that defines a first locking part and an open end. The first section is disposed adjacent to the open end such that the first section and the intermediate section define a first transition defining a first face. The second section is disposed adjacent to the open end such that the second section and the intermediate section define a second transition defining a second face, wherein the first face is angularly disposed relative to the second face. A resistance member has an exterior surface that defines a second locking part configured for engagement with the first locking part such that the resistance member is fixed with and engaging at least a portion of the inner surface.

The intermediate section can extend from the first and second transitions such that the intermediate section is offset from the first and second sections. The intermediate section may have a C-shaped configuration defining a correspondingly shaped inner surface and the open end, whereby the resistance member is configured to prevent closing of the open end.

### BRIEF DESCRIPTION OF THE DRAWINGS

The embodiments of Figs. 10, 14, 44-47 and 54 as such do not form embodiments of the invention, however, help understanding aspects of the invention.

The present disclosure will become more readily apparent from the specific description accompanied by the following drawings, in which:
FIG. 1 is a perspective view of one particular embodiment of the vertebral rod system in accordance with the principles of the present disclosure;
FIG. 2 is a perspective view of a vertebral rod of the vertebral rod system shown in FIG. 1;
FIG. 3 is a side plan view of the vertebral rod shown in FIG. 2;
FIG. 4 is a perspective view of a resistance member of the vertebral rod system shown in FIG. 1;
FIG. 5 is a side, cross-section view of the resistance member taken along line 5-5 in FIG. 4;
FIG. 6 is a perspective view of a vertebral rod system of the present disclosure attached to vertebrae;
FIG. 7 is a lateral section view of the vertebral rod system of the present disclosure attached to vertebrae illustrating rod movement;
FIG. 8 is a side view of an alternate embodiment of the vertebral rod shown in FIG. 2;
FIG. 9 is a front view of another alternate embodiment of the vertebral rod shown in FIG. 2;
FIG. 10 is a side view of another alternate embodiment of the vertebral rod shown in FIG. 2;
FIG. 11 is a lateral section view of an alternate embodiment of the vertebral rod system employing the vertebral rod shown in FIG. 10 attached to vertebrae;
FIG. 12 is a side view of another alternate embodiment of the vertebral rod shown in FIG. 2;
FIG. 13 is a front view of an alternate embodiment of the vertebral rod shown in FIG. 11;
FIG. 14 is a side view of another alternate embodiment of the vertebral rod shown in FIG. 2;
FIG. 15 is a side view of another alternate embodiment of the vertebral rod shown in FIG. 2;
FIG. 16 is a side view of another alternate embodiment of the vertebral rod shown in FIG. 2;
FIG. 17 is a perspective view of another alternate embodiment of the vertebral rod shown in FIG. 2;
FIG. 18 is a perspective view of an alternate embodiment of the resistance member shown in FIG. 4;
FIG. 19 is a perspective view of another alternate embodiment of the resistance member shown in FIG. 4;
FIG. 20 is a perspective view of another alternate embodiment of the resistance member shown in FIG. 4;
FIG. 21 is a perspective view of another alternate embodiment of the resistance member shown in FIG. 4;
FIG. 22 is a lateral section view of an alternate embodiment of the vertebral rod system attached to vertebrae;
FIG. 23 is a side view of an alternate embodiment of the vertebral rod system employing the vertebral rod shown in FIG. 8;
FIG. 24 is a front view of the vertebral rod system shown in FIG. 23;
FIG. 25 is a side view of an alternate embodiment of the vertebral rod system employing the vertebral rod shown in FIG. 8;
FIG. 26 is a front view of the vertebral rod system shown in FIG. 25;
FIG. 27 is a side view of an alternate embodiment of the vertebral rod system employing the vertebral rod shown in FIG. 8;
FIG. 28 is a front view of the vertebral rod system shown in FIG. 27;
FIGS. 29 - 43 are side views of alternate embodiments of locking parts of the vertebral rod system, in accordance with the principles of the present disclosure;
FIG. 44 is a side perspective view of another alternate embodiment of the vertebral rod shown in FIG. 2;
FIG. 45 is a side cross-section view of the vertebral rod taken along line 45-45 shown in FIG. 44;
FIG. 46 is a front cross-section view of the vertebral rod taken along line 46-46 shown in FIG. 44;
FIG. 47 is a side view of another alternate embodiment of the vertebral rod shown in FIG.2;
FIG. 48 is a side view of another alternate embodiment of the vertebral rod shown in FIG. 2;
FIG. 49 is a front view of the vertebral rod shown in FIG. 48;
FIG. 50 is a cross section view of an alternate embodiment of the resistance member shown in FIG. 4;
FIG. 51 is a side view of another alternate embodiment of the vertebral rod shown in FIG. 2;
FIG. 52 is a front view of the vertebral rod shown in FIG. 51;
FIG. 53 is a side, enlarged cutaway view of the vertebral rod shown in FIG. 51; and
FIGS. 54A-H are cross-section views of alternate embodiments of locking parts of the vertebral rod system, in accordance with the principles of the present disclosure.

Like reference numerals indicate similar parts throughout the figures.

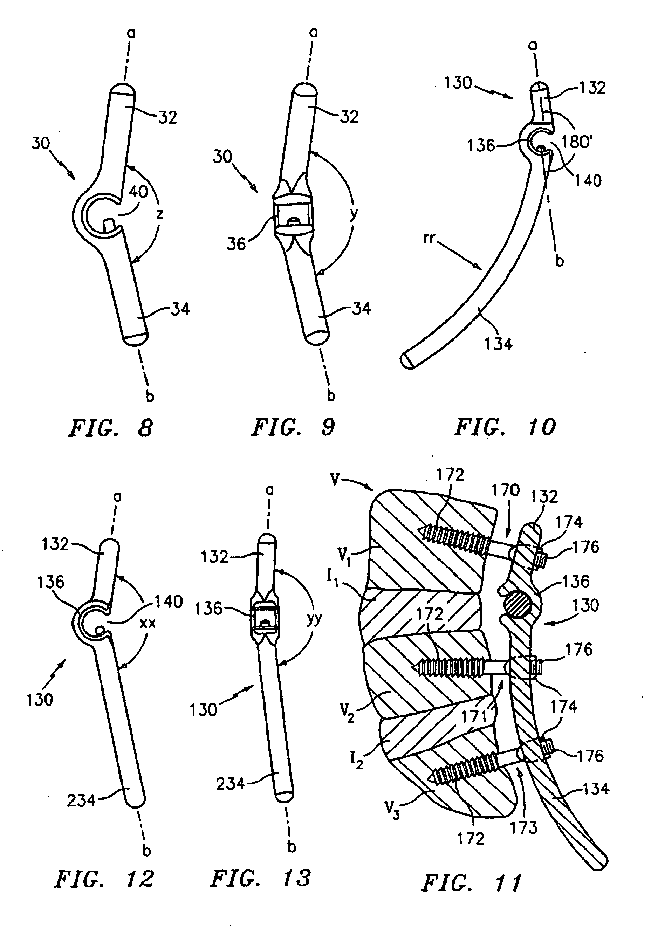

Figures 10, 14 and 44 to 47 do not show embodiments of the invention as such, but show embodiments that help understanding aspects of the invention.

### DETAILED DESCRIPTION OF THE INVENTION

The exemplary embodiments of the vertebral rod system and methods of use disclosed are discussed in terms of medical devices for the treatment of spinal disorders and more particularly, in terms of a dynamic vertebral rod system having flexion and extension capability. It is envisioned that the vertebral rod system and methods of use disclosed provide stability and maintains structural integrity while reducing stress on spinal elements. It is envisioned that the preset dislosure may be employed to treat spinal disorders such as, for example, degenerative disc disease. disc herniation, osteoporosis, spondylolisthesis, stenosis, scoliosis and other curvature abnormalities, kyphosis, tumor and fractures. It is further envisioned that the present disclosure may be employed with surgical treatments including open surgery and minimally invasive procedures, of such disorders, such as, for example, discectomy, laminectomy, fusion, bone graft and implantable prosthetics. It is contemplated that the present disclosure may be employed with other osteal and bone related applications, including those associated with diagnostics and therapeutics. It is further contemplated that the disclosed vertebral rod system may be employed in a surgical treatment with a patient in a prone or supine position, employing a posterior, lateral or anterior approach. The present disclosure may be employed with procedures for treating the lumbar, cervical, thoracic and pelvic regions of a spinal column.

The present invention may be understood more readily by reference to the following detailed description of the invention taken in connection with the accompanying drawing figures, which form a part of this disclosure. It is to be understood that this invention is not limited to the specific devices, methods, conditions or parameters described and/or shown herein, and that the terminology used herein is for the purpose of describing particular embodiments by way of example only and is not intended to be limiting of the claimed invention. Also, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment.

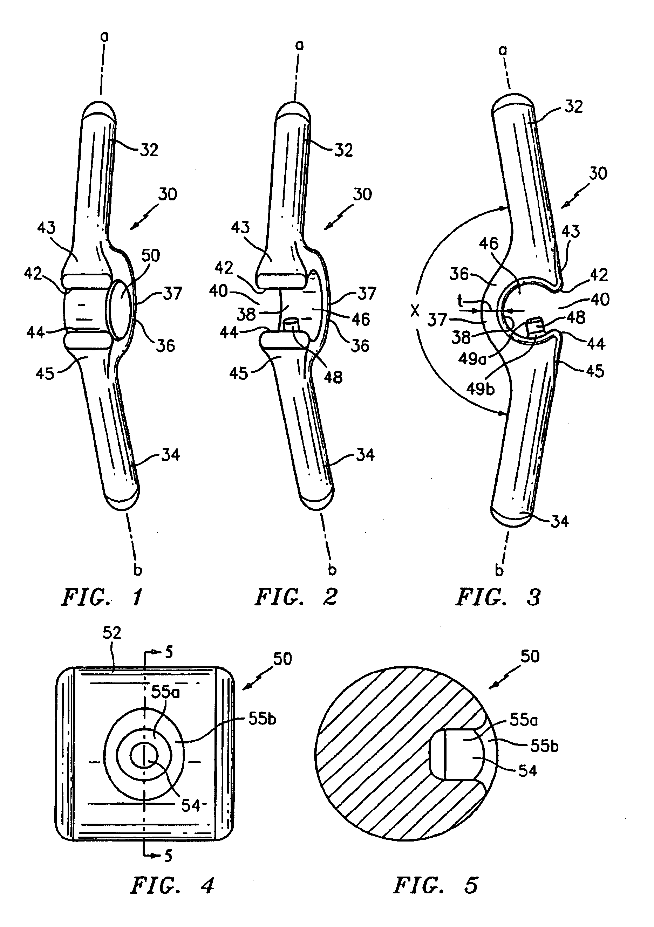

The following discussion includes a description of a vertebral rod system, related components and exemplary methods of employing the vertebral rod system in accordance with the principles of the present disclosure. Alternate embodiments are also disclosed. Reference will now be made in detail to the exemplary embodiments of the present disclosure, which are illustrated in the accompanying figures. Turning now to FIGS. 1-5, there is illustrated components of a vertebral rod system in accordance with the principles of the present disclosure.

The components of the vertebral rod system are fabricated from materials suitable for medical applications, including metals, polymers, ceramics, biocompatible materials and/or their composites, depending on the particular application and/or preference of a medical practitioner. For example, a vertebral rod, discussed below, of the vertebral rod system can be fabricated from materials such as titanium, thermoplastics such as polyaryletherketone (PAEK) including PEEK, PEKK and PEK, carbon-PEEK composites, PEEK-BaSO₄ polymeric rubbers, biocompatible materials such as polymers including plastics, metals, ceramics and composites thereof, rigid polymers including polyphenylene, polyamide, polyimide, polyetherimide, polyethylene, epoxy; and different sections of the rod may have alternative material composites to achieve various desired characteristics such as strength, rigidity, elasticity, compliance biomechanical performance, durability and radiolucency or imaging preference.

For example, the vertebral rod can be formed of two or more materials. In one embodiment, elongated rod sections can be fabricated from carbon-reinforced PEEK and an intermediate section can be fabricated from PEEK. In another embodiment, elongated rod sections are fabricated from PEEK and an intermediate section is fabricated from carbon-reinforced PEEK. In another embodiment, alternate materials may be employed in a radial direction of a vertebral rod such that stiff materials such as metals or other composites are used in a core of the rod sections and an outer sheet of lower modulus polymeric material is used in the outer radial portion of the rod sections, or vice versa. In another embodiment employing a composite material similar to those described, the elongated rod sections can have a cylindrical geometry and the intermediate section can have a rectangular or oblong geometry.

As a further example, a resistance member of the vertebral rod system may be fabricated from materials such as silicone, polyurethane, silicone-polyurethane copolymers, polymeric rubbers, polyolefin rubbers, hydrogels, semirigid and rigid materials, and biocompatible materials such as elastomers, rubbers, thermoplastic elastomers, thermoset elastomers, elastomeric composites and plastics. It is envisioned that the rod sections can be manufactured from, for example, machining and milling from a solid stock material and/or injection molding. The resistance member can be manufactured from, for example, machining and milling, extrusion and die cutting, injection molding, transfer molding and/or cast molding. One skilled in the art, however, will realize that such materials and fabrication methods suitable for assembly and manufacture, in accordance with the present disclosure, would be appropriate.

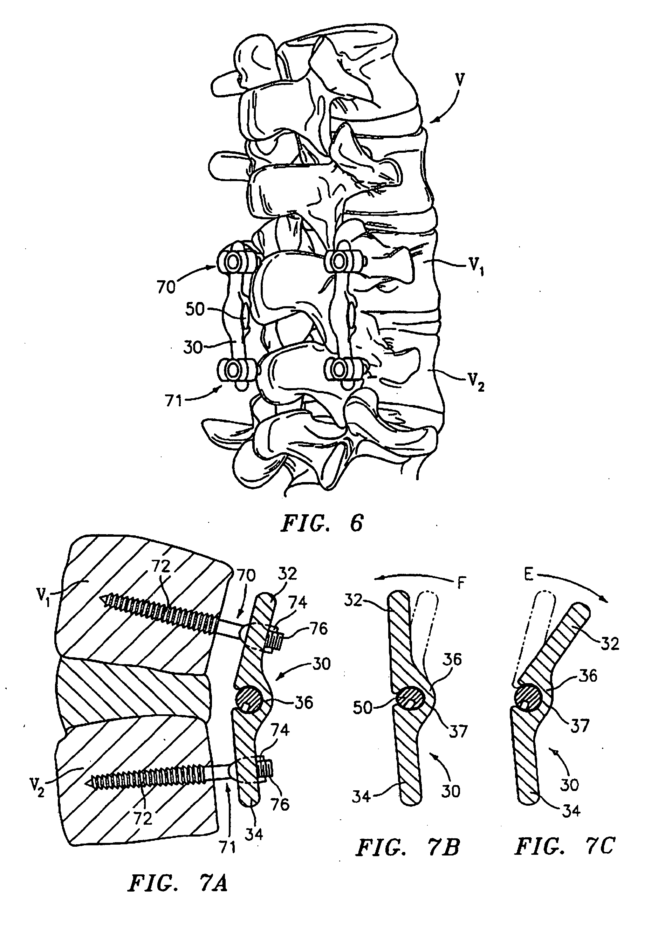

The vertebral rod system is configured for attachment to vertebrae (as shown, for example, in FIG. 6) during surgical treatment of a spinal disorder, examples of which are discussed herein. The vertebral rod system has a vertebral rod 30, which includes a first elongated section, such as, for example, upper section 32 that defines a longitudinal axis *a*. A second elongated section, such as, for example, lower section 34 defines a longitudinal axis *b*.

An intermediate section 36 is connected with sections 32, 34 and disposed therebetween as a joining section of the components of vertebral rod 30. It is envisioned that the components of vertebral rod 30 may be monolithically formed, integrally connected or arranged with attaching elements. Intermediate section 36 is flexible relative to sections 32, 34, and is configured to provide resistance to movement of sections 32, 34. It is envisioned that intermediate section 36 may provide increasing, variable, constant and/or decreasing resistance. It is contemplated that sections 32, 34, 36 can be variously dimensioned, for example, with regard to length, width, diameter and thickness. It is further contemplated that the respective cross-section of sections 32, 34, 36 may have various configurations, for example, round, oval, rectangular, irregular, uniform and non-uniform. Section 32 may have a different cross-sectional area, geometry, material or material property such as strength, modulus or flexibility relative to section 34.

Intermediate section 36 may have a variable thiekness *t* (FIG. 3) according to the requirements of the particular application. It is envisioned that thickness *t* of intermediate section 36 may be in a range of 1-10 mm, preferably in a range of 2-8 mm, and most preferably in a range of 3-5 mm. It is further envisioned that the cross-sectional geometry or area of intermediate section 36 can be uniform, non-uniform, consistent or variable.

It is envisioned that intermediate section 36 may be configured as a flexible joint having a wide, narrow, round or irregular configuration. It is further envisioned that intermediate section 36 can be variously configured and dimensioned with regard to size, shape, thickness, geometry and material. Intermediate section 36 may also have one or a plurality of elements connecting sections 32, 34 such as spaced apart portions, staggered patterns and mesh. Intermediate section 36 may be fabricated from the same or alternative material to sections 32, 34. Intermediate section 36 may also have a different cross-sectional area, geometry or material property such as strength, modulus and flexibility relative to sections 32, 34. Intermediate section 36 may be connected to sections 32, 34 using various methods and structure including molding of a continuous component, mechanical fastening, adhesive bonding and combinations thereof. It is envisioned that intermediate section 36 has a flexible hinge configuration, which can be offset forward or backward relative to a central axis of rod 30 to modify the flexibility or stiffness of the vertebral rod system. It is further envisioned that particular parameters may be selected to modulate the flexibility or stiffness of the vertebral rod system including the cross-sectional area (or thickness) of intermediate section 36, material modulus that may correlate to the hardness of bumper 50 discussed below, modification of porosity in a range of 0-30 percent which may include modification of void volume in a range of 10 microns - 1mm, as well as rod material properties. These parameters allow modification of the properties or performance of the vertebral rod system such as strength, durability, flexibility (or stiffness), overall profile and the ability to employ a percutaneous approach, for a particular application.

intermediate section 36 includes a flexible joint member 37, which has a C-shaped configuration and defines a corresponding shaped arcuate inner surface 38 and an open end 40. It is further contemplated that vertebral rod 30 may include one or a plurality of intermediate sections 36 spaced along the length of rod 30. In embodiments including a plurality of sections 36, the multiple sections 36 may be disposed in similar, or alternative orientations such as aligned, non-aligned, offset, open end facing or not facing vertebrae and alternate angular orientation.

Upper section 32 is disposed adjacent to an upper portion 42 of open end 40 and the transition defines a front face 43. Lower section 34 is disposed adjacent a lower portion 44 and the transition defines a front face 45. Inner surface 38 defines a cavity 46 and a first locking part, such as, for example, a post 48. Post 48 has a first portion 49a, which is cylindrical, and a second portion 49b, which has an increasing diameter as post 48 transitions into surface 38, as shown in FIG. 3.

Cavity 46 is configured for disposal of a resistance member, such as, for example, a bumper 50, as shown in FIGS. 4 and 5. Bumper 50 has an exterior surface 52 that defines a second locking part, such as, for example, an opening 54. Opening 54 has a first portion 55a configured for receipt of portion 49a, and a second portion 55b having an increasing diameter and being configured for receipt of portion 49b. Opening 54 receives post 48 for fixed mounting of bumper 50 with vertebral rod 30 to lock these components of the vertebral rod system in place. It is contemplated that portions 49a, 49b may be variously configured and dimensioned, and portions 55a, 55b correspondingly configured and dimensioned for reception thereof. Portions 49a, 49b may be uniform in configuration and dimension. It is envisioned that the first locking part may include one or a plurality of elements, may be variously disposed about intermediate section 36, or employ fastening elements and adhesives, with the second locking part being correspondingly configured for engagement therewith.

Bumper 50 is elastic and configured to provide variable resistance to movement of sections 32, 34 and 36. It is contemplated that bumper 50 can provide increasing, variable, constant and/or decreasing resistance. Bumper 50 is disposed within cavity 46 and engages surface 38 in a close fitting engagement. Bumper 50 can be variously configured with regard to size, shape, for example, round, oblong, spherical. It is envisioned that bumper 50 has a hardness in the range of 20 Shore A to 55 Shore D, and preferably between 70 and 90 Shore A. The material of bumper 50 can be solid or porous, homogeneous or heterogeneous, single polymer or a blend/composite of more than one polymer. It is contemplated that the resiliency of bumper 50 can prevent creep and improve shape recovery of the vertebral rod system. It is envisioned that bumper 50 is configured to prevent and/or resist closing of open end 40. It is further envisioned that bumper 50 is secured in place with intermediate section 36, and desirably mechanically secured therewith in a configuration to present migration and expulsion therefrom. In other embodiments, bumper 50 can be textured, encapsulated, adhesively bonded and/or over molded with vertebral rod 30. Bumper 50 can be inserted with cavity 46 for assembly, or formed in situ by, for example, a pouch, bag or balloon with the bumper configuration being inserted into cavity 46 and injected with a curable material.

In a first orientation of vertebral rod 30, longitudinal axis *a* is disposed at an angle *x* relative to longitudinal axis *b* about joint member 37, as shown in FIG. 3. Angle *x* is desirably in a range of 135 degrees to less than 180 degrees, and most desirably in a range of 150 degrees to 160 degrees. Angle *x* may be equal to 180 degrees. It is contemplated that in the first orientation, no flexion or extension forces are applied to vertebral rod 30. As sections 32, 34, 36 move to a second orientation from the first orientation, flexion and/or extension forces are applied to vertebral rod 30. As such, bumper 50 engagingly interacts with intermediate section 36 in a configuration that provides increasing resistance to movement of sections 32, 34 from the first orientation to the second orientation. Movement of the components of the vertebral rod system between one or a plurality of orientations is contemplated and may include a range of increasing and decreasing levels of resistance of the components of the vertebral rod system.

In assembly, operation and use, the vertebral rod system is employed with a surgical procedure for treatment of a spinal disorder affecting a section of a spine of a patient, as discussed herein. The vertebral rod system may also be employed with other surgical procedures. In particular, the vertebral rod system is employed with a surgical procedure for treatment of a condition or injury of an affected section of the spine including vertebrae V, as shown in FIGS. 6 and 7. It is contemplated that the vertebral rod system is attached to vertebrae V for dynamic stabilization of the affected section of the spine to facilitate healing and therapeutic treatment, while providing flexion and extension capability.

In use, to treat the affected section of the spine, a medical practitioner obtains access to a surgical site including vertebra V in any appropriate manner, such as through incision and retraction of tissues. It is envisioned that the vertebral rod system may be used in any existing surgical method or technique including open surgery, mini-open surgery, minimally invasive surgery and percutaneous surgical implantation, whereby the vertebrae V is accessed through a micro-incision, or sleeve that provides a protected passageway to the area. Once access to the surgical site is obtained, the particular surgical procedure is performed for treating the spinal disorder. The vertebral rod system is then employed to augment the surgical treatment. The vertebral rod system can be delivered or implanted as a pre-assembled device or can be assembled in situ. The vertebral rod system may be completely or partially revised, removed or replaced, for example, replacing bumper 50 only, replacing rod 30 and bumper 50 and using the in-place fastening elements.

A first fastening element, such as, for example, fixation screw assembly 70 is configured to attach upper section 32 to vertebra V₁. A second fastening element, such as, for example, fixation screw assembly 71 is configured to attach lower section 34 to adjacent vertebra V₂. Pilot holes are made in vertebrae V₁, V₂ for receiving fixation screw assemblies 70, 71. Fixation screw assemblies 70, 71 include threaded bone engaging portions 72 that are inserted or otherwise connected to vertebrae V₁, V₂, according to the particular requirements of the surgical treatment. Fixation screw assemblies 70, 71 each have a head 74 with a bore, or through opening and a set screw 76, which is torqued on to sections 32, 34 to attach rod 30 in place with vertebrae V, as will be described.

As shown in FIG. 6, the vertebral rod system includes two axially aligned and spaced rods 30, with portions of sections 32, 34 extending through the bores of heads 74. Set screws 76 of each head 74 are torqued on the end portions of rods 30 to securely attach rods 30 with vertebrae V₁, V₂. Upon fixation of the vertebral rod system with vertebrae V, vertebral rod 30 is configured to provide increasing resistance to movement of sections 32, 34 during flexion and extension of the spine. For example, vertebral rod 30, as shown in FIG. 7A, is in an unloaded state, which corresponds to the first orientation discussed above, where there is no appreciable tensile or compressive loads on vertebrae V₁, V₂. In flexion and/or extension of vertebrae V caused by corresponding movement of the patient, rod 30 reacts with increasing resistance during movement of rod 30 to a second, third or more orientation(s).

In flexion, as shown in FIG. 7B, upper section 32 moves relative to section 34, in the direction of arrow F. Joint member 37 flexibly expands circumferentially about bumper 50 such that intermediate section 36 compresses bumper 50. This configuration increases resistance during flexion. In extension, as shown in FIG. 7C, upper section 32 moves relative to section 34, in the direction shown by arrow E. Joint member 37 flexibly compresses circumferentially about bumper 50. Inner surface 38 adjacent bumper 50 is in tension and the opposing edge of joint member 37 is in compression such that joint member 37 does not significantly compress bumper 50. Resistance is increased during extension. The increase of resistance during flexion and extension provides limited movement of vertebrae V for dynamic stabilization of the treated area of the spine.

The vertebral rod system can be used with various bone screws, pedicle screws or multi-axial screws (MAS) used in spinal surgery. It is contemplated that the vertebral rod system may be used with pedicle screws coated with an osteoconductive material such as hydroxyapatite and/or osteoinductive agent such as a bone morphogenic protein for enhanced bony fixation to facilitate motion of the treated spinal area. Rod 30 and bumper 50 can be made of radiolucent materials such as polymers. Radiomarkers may be included for identification under x-ray, fluoroscopy, CT or other imaging techniques. Metallic or ceramic radiomarkers, such as tantalum beads, tantalum pins, titanium pins, titanium endcaps and platinum wires can be used, such as being disposed at the end portions of rod 30 and/or along the length thereof adjacent joint member 37 or with bumper 50.

Referring to FIG. 8, in an alternate embodiment of vertebral rod 30, similar to that described with regard to FIGS. 1-3, upper section 32 and lower section 34 are disposed in an orientation such that longitudinal axis *a* is disposed at an angle *z* relative to longitudinal axis *b* about open end 40. Angle *z* is desirably in a range of 135 degrees to less than 180 degrees, and most desirably in a range of 150 degrees to 160 degrees. Angle z may be equal to 180 degrees. Referring to FIG. 9, in another alternate embodiment of vertebral rod 30, similar to that described, upper section 32 and lower section 34 are disposed in a laterally offset orientation such that longitudinal axis *a* is disposed at an angle *y* relative to longitudinal axis *b* about the side of intermediate section 36. Angle y is desirably in a range of 135 degrees to less than 180 degrees, and most desirably in a range at 150 degrees - 160 degrees. Angle y may be equal to 180 degrees. It is contemplated that the vertebral rod system may be disposed in an angular orientation according to the particular angle *z* and angle *y* such that rod 30 may offset both axially and laterally.

Referring to FIG. 10, an alternate embodiment of the vertebral rod system includes a vertebral rod 130, similar to vertebral rod 30 described with regard to FIGS. 1-3. Vertebral rod 130 includes an upper section 132, an intermediate section 136 and a lower section 134, similar to those sections described above. Upper section 132 has a first length and lower section 134 has a second, greater length. In a first orientation of vertebral rod 130, longitudinal axis *a* is disposed at an angle of 180 degrees relative to longitudinal axis *b*, about an open end 140. It is contemplated that longitudinal axis a may be disposed at other angular orientations relative to longitudinal axis *b*, including those discussed herein.

Lower section 134 has an arcuate configuration and an increased length providing the ability to extend over two or more intervertebral elements. It is contemplated that the configuration of the vertebral rod system may provide dynamic or flexible stabilization over a plurality of intervertebral levels, including treated and untreated vertebral and intervertebral levels. It is further contemplated that lower section 134 provides a less flexible, or more rigid stabilization relative to upper section 132 and intermediate section 136. It is envisioned that lower section 134 may be attached with vertebrae across lower lumbar levels such as levels L5-S1. Lower section 134 may be cut or trimmed during a surgical procedure such that the size of vertebral rod 130 can be modified according to patient needs or the particular requirements of a surgical treatment or medical practitioner.

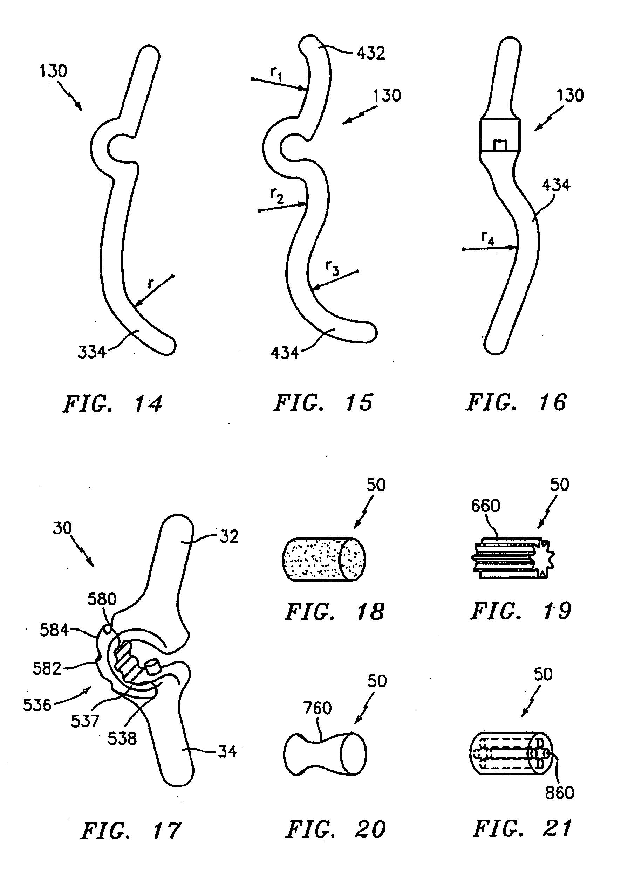

The arcuate configuration of lower section 134 has a radius of curvature *rr*. Desirably, the radius of curvature *rr* is in a range of 20-400 mm, preferably in a range of 50-200 mm, and most preferably in a range of 100-150 mm. In an alternate embodiment, upper section 132 can have an arcuate configuration and/or an increased length, similar to that described. An arcuately configured upper section 132 has a radius of curvature including those ranges discussed herein. It is contemplated that the arcuately configured section 132 may have an equivalent or non-equivalent radius, same or alternate orientation relative to lower section 134. It is further contemplated that upper section 132 may include a laterally offset orientation, similar to that discussed with regard to FIGS. 9 and 16.

Referring to FIG. 11, an alternate embodiment of the method of use of the vertebral rod system with a surgical procedure for treating a spinal disorder, similar to that described with regard to FIGS. 6 and 7, includes vertebral rod 130 discussed above. The vertebral rod system includes fixation screw assemblies

170, 171 and 173, which include threaded bone engaging portions 172 that are inserted or otherwise connected to vertebrae V₁, V₂ and V₃, according to the particular requirements of the surgical treatment. Fixation screw assemblies 170, 171 and 173 each have a head 174 with a through opening and a set screw 176, which is torqued on to vertebral rod 130 to attach rod 130 in place with vertebrae V.

Upper section 132 has a shorter rod length, relative to lower section 134. Fixation screw assembly 170 is torqued on to upper section 132 for attachment with vertebra V₁. Lower section 134 has a longer rod length that extends across intervertebral disc elements I₁ and I₂. Fixation screw assemblies

171, 173 are torqued on to lower section 134 for attachment with vertebrae V₂, V₃. Motion is preserved while providing stability to an untreated intervertebral level.

It is envisioned that upper section 132 and intermediate section 136 are used for lumbar levels such as L4-L5. It is contemplated that lower section 134 is used for a lower lumbar level, such as L5-S1. It is contemplated that vertebral rod 130 is configured such that lower section 134 can be cut or trimmed as desired during the surgical procedure. It is envisioned that the vertebral rod may be heat treated during surgery to obtain a best fit curvature or shape for the patient. It is further envisioned that vertebral rod 130 may include one or a plurality of intermediate sections 136 spaced along the length of rod 130, such as, for example, an additional section 136 being disposed between fixation screw assemblies 171 and 173. In embodiments including a plurality of sections 136, the multiple sections 136 may be disposed in similar, or alternative orientations such as aligned, non-aligned, offset, open end facing or not facing vertebrae and alternate angular orientation.

Referring to FIG. 12, in an alternate embodiment, vertebral rod 130 has a linearly configured lower section 234. In a first orientation of vertebral rod 130, upper section 132 defines longitudinal axis *a,* which is disposed at an angle *xx* relative to a longitudinal axis *b* of lower section 234, about an open end 140 of intermediate section 136. Angle *xx* is desirably in a range of 135 degrees to less than 180 degrees, and most desirably in a range of 150 degrees to 160 degrees. Angle *xx* may be equal to 180 degrees.

Referring to FIG. 13, in another alternate embodiment of vertebral rod 130, similar to that described with regard to FIG. 12, upper section 132 and lower section 234 are disposed in a laterally offset orientation such that axis a is disposed at angle *yy* relative to longitudinal axis *b* about the side of intermediate section 136. Angle *yy* is desirably in a range of 135 degrees to less than 180 degrees, and most desirably in a range of 150-160 degrees. Angle *yy* may be equal to 180 degrees. It is contemplated that the vertebral rod system may be disposed in an angular orientation according to the particular angle *xy* and angle *yy* such that rod 130 may offset both axially and laterally.

Referring to FIG. 14, in an alternate embodiment of vertebral rod 130, similar to that described with regard to FIG. 11, a lower section 334 has an arcuate configuration with a corresponding radius of curvature *r.* Desirably, the radius of curvature *r* is in a range of 20-400 mm, preferably in a range of 50-200 mm, and most preferably in a range of 100-150 mm.

Referring to FIG. 15, in another alternate embodiment of vertebral rod 130, similar to those described above, an upper section 432 has an arcuate configuration with a corresponding radius of curvature *r₁*. Desirably, the radius of curvature *r₁* is in a range of 20-400 mm, preferably in a range of 50-200 mm, and most preferably in a range of 100-150 mm. A lower section 434 has an undulating configuration with corresponding radii of curvature *r₂, r₃.* Radii *r₂, r₃* are desirably in a range of 20-400 mm, preferably in a range of 50-200 mm, and most preferably in a range of 100-150 mm, and may be of equal value, non-equivalent or zero.

Referring to FIG. 16, in another alternate embodiment of vertebral rod 130 shown in FIG. 15, lower section 434 includes a laterally oriented curvature with a corresponding radius of curvature *r₄,* which is desirably in a range of 20-400 mm, preferably in a range of 50-200 mm, and most preferably in a range of 100-150 mm.

Referring to FIG. 17, in an alternate embodiment of vertebral rod 30, similar to that described with regard to FIGS. 1-3, an intermediate section 536 has an inner surface 538 that includes a plurality of grooves 580. Grooves 580 are transversely disposed about the circumference of inner surface 538. Intermediate section 538 has an exterior surface 582, which includes a plurality of grooves 584. Grooves 584 are transversely disposed about joint member 537. It is contemplated that one or a plurality of grooves may be defined in surfaces 538, 582. It is further contemplated that grooves 580, 584 may be oriented longitudinally. Grooves 580, 584 may be disposed on only one of surface 538 or surface 582. It is envisioned that the grooves may be staggered or discontinuous.

Referring to FIG. 18, in an alternate embodiment, bumper 50 is fabricated from a porous or foam material. In another alternate embodiment, bumper 50 has a gear surface configuration including teeth 660, as shown in FIG. 19. In another alternate embodiment, bumper 50 has a dumbbell configuration including elliptical surfaces 760, as shown in FIG. 20. In another alternate embodiment, bumper 50 has through holes 860, as shown in FIG. 21.

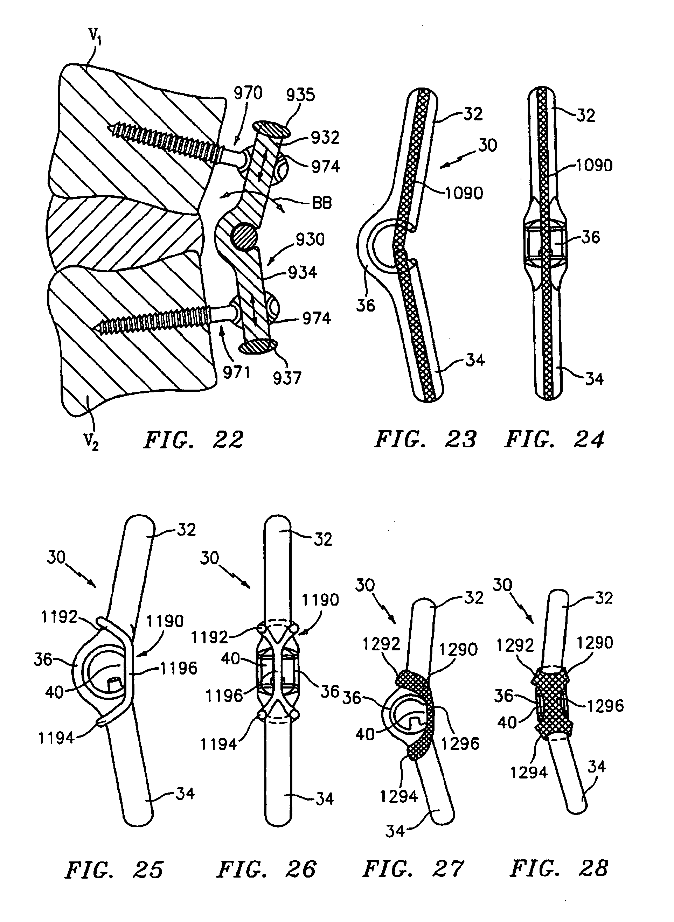

Referring to FIG. 22, in alternate embodiment of the vertebral rod system employing components similar to those described above, fixation screw assemblies 970, 971, similar to assemblies 70, 71, described above, are employed for attaching a vertebral rod 930, similar to rod 30 described above, to vertebrae *V*₁*, V*₂*.* Fixation screw assemblies 970, 971 include heads 974 configured for relative movement of rod 930 therein. Rod 930 includes an upper section 932 and a lower section 934, which are relatively moveable within the respective through openings of heads 974. Upper section 932 includes a stop 935 defined at an end portion thereof. Lower section 934 includes a stop 937 defined at an end portion thereof. Stops 935, 937 are configured to prevent disengagement of vertebral rod 930 from fixation screw assemblies 970, 971 during movement of vertebrae V₁, V₂ under flexion and extension. It is envisioned that assemblies 970, 971 include non-locking multi axial screws such that sections 932, 934 freely slide under applied tensile and compressive loads in connection with the flexion/extension of rod 930. Sections 932, 934 may include an elongated stop or end cap to limit sliding or further prevent rod 930 from slipping out of engagement with fixation screw assemblies 970, 971. Sections 932, 934 may also be torqued for fixation with set screws or the like.

Referring to FIGS. 23 and 24, in another alternate embodiment of the vertebral rod system including vertebral rod 30, similar to that described with regard to FIG. 8, a tension element, such as, for example, a band 1090 is disposed about upper section 32, lower section 34 and intermediate section 36 in a configuration to limit movement of sections 32, 34 from the first orientation. Band 1090 can be secured to ends of sections 32, 34 with crimp, attached lockcap, loop around a pin or tied knot. Band 1090 may include a tether or a cable and is desirably fabricated from an elastic material. Band 1090 augments resistance of rod 30 with regard to movement of sections 32, 34 in flexion/extension, as described above.

Referring to FIGS. 25 and 26, in an alternate embodiment of the vertebral rod system shown in FIGS. 23 and 24, a band 1190 includes a loop 1192 disposed about upper section 32 and a loop 1194 disposed about lower section 34. A central portion 1196 of band 1190 is disposed across open end 40. Referring to FIGS. 27 and 28, in another alternate embodiment of the vertebral rod system shown in FIGS. 23 and 24, a band is configured as a woven mesh 1290. Mesh 1290 includes a loop 1292 disposed about upper section 32 and a loop 1294 disposed about lower section 34. A central portion 1296 is disposed across open end 40. It is contemplated that mesh 1290 is fabricated from an elastic material.

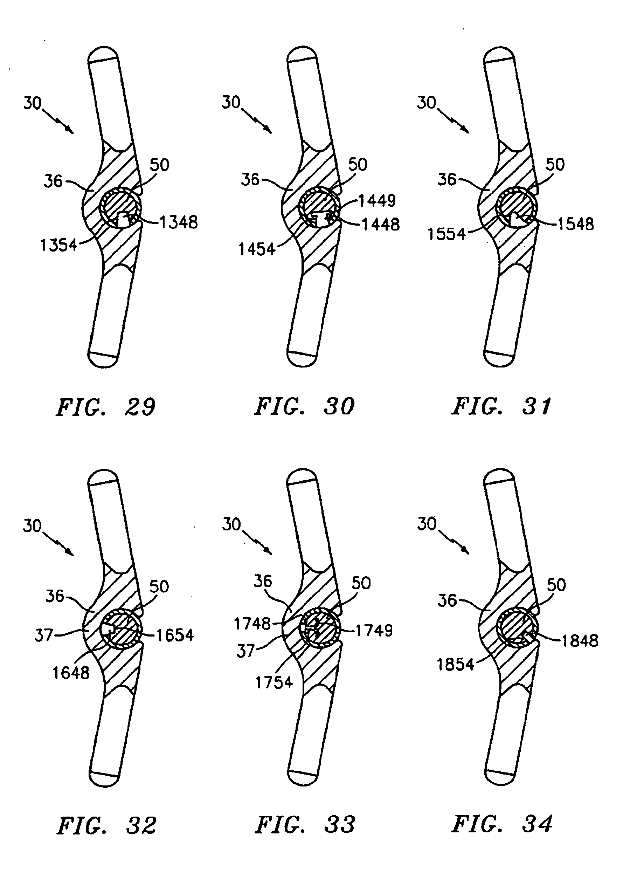

Referring to FIGS. 29-43, the vertebral rod system can include alternate embodiments of the locking parts of intermediate section 36 and bumper 50, similar to that described with regard to FIGS. 1-3. As shown in FIG. 29, intermediate section 36 includes a first locking part, a conical shaped post 1348 and bumper 50 includes a second locking part, an opening 1354 configured for reception thereof and locking engagement of bumper 50 with vertebral rod 30. Alternatively, as shown in FIG. 30, intermediate section 36 includes a first locking part, a post 1448 having a barb 1449 and bumper 50 includes a second locking part, an opening 1454 configured for reception thereof and locking engagement of bumper 50 with vertebral rod 30. Alternatively, as shown in FIG. 31, intermediate section 36 includes a first locking part, a wedge shaped post 1548 and bumper 50 includes a second locking part, an opening 1554 configured for reception thereof and locking engagement of bumper 50 with vertebral rod 30.

Alternatively, as shown in FIG. 32, intermediate section 36 includes a first locking part, a conical shaped post 1648 disposed with joint member 37 and bumper 50 includes a second locking part, an opening 1654 configured for reception thereof and locking engagement of bumper 50 with vertebral rod 30. Alternatively, as shown in FIG. 33, intermediate section 36 includes a first locking part, a post 1748 having a dual hook 1749, disposed with joint member 37, and bumper 50 includes a second locking part, an opening 1754 configured for reception thereof and locking engagement of bumper 50 with vertebral rod 30. Alternatively, as shown in FIG. 34, intermediate section 36 includes a first locking part, a pin shaped post 1848 and bumper 50 includes a second locking part, an opening 1854 configured for reception thereof and locking engagement of bumper 50 with vertebral rod 30.

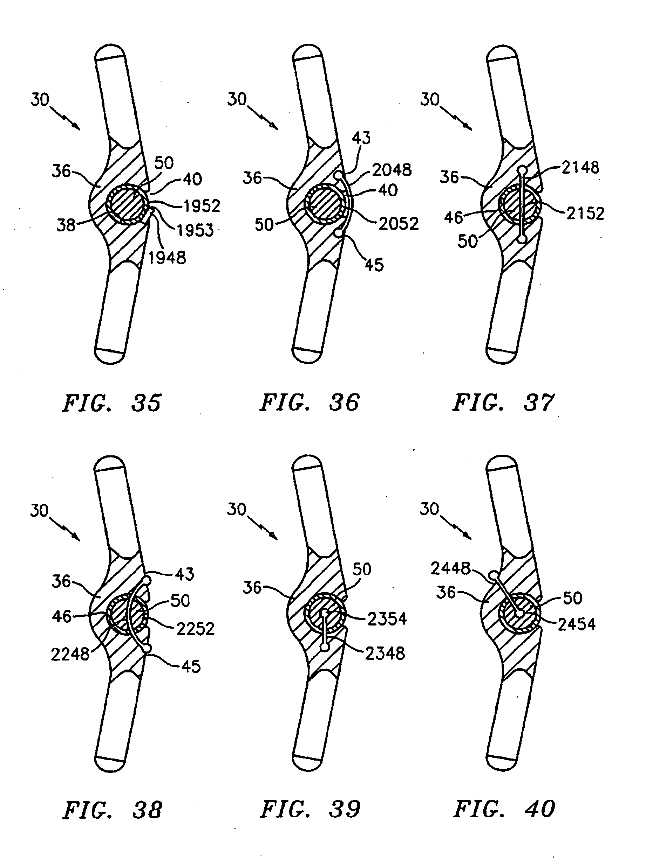

Alternatively, as shown in FIG. 35, intermediate section 36 includes a first locking part, a post 1948 extending from inner surface 38 and across a portion of open end 40. Bumper 50 includes a second locking part, an exterior surface 1952 having a recess 1953 configured for reception of post 1948 and locking engagement of bumper 50 with vertebral rod 30. Alternatively, as shown in FIG. 36, intermediate section 36 includes a first locking part, a tether 2048 connected adjacent faces 43, 45 and extending across open end 40. Bumper 50 includes an exterior surface 2052 configured for engagement with tether 2048 such that bumper 50 is fixed with vertebral rod 30. Alternatively, as shown in FIG. 37, intermediate section 36 includes a first locking part, a tether 2148 connected adjacent a side portion thereof and extending across a lateral open portion of cavity 46. Bumper 50 includes an exterior surface 2152 configured for engagement with tether 2148 such that bumper 50 is fixed with vertebral rod 30.

Alternatively, as shown in FIG. 38, intermediate section 36 includes a first locking part, a tether 2248 connected adjacent faces 43, 45 and extending across a lateral open portion of cavity 46. Bumper 50 includes an exterior surface 2252 configured for engagement with tether 2248 such that bumper 50 is fixed with vertebral rod 30. Alternatively, as shown in FIG. 39, intermediate section 36 includes a first locking part, a tethered connection 2348 disposed on a lower side portion thereof. Bumper 50 includes a second locking part, a tethered connection 2354 in a tethered configuration with intermediate section 36 for locking engagement of bumper 50 with vertebral rod 30. Alternatively, as shown in FIG. 40, intermediate section 36 includes a first locking part, a tethered connection 2448 disposed on an upper side portion thereof. Bumper 50 includes a second locking part, a tethered connection 2454 in a tethered configuration with intermediate section 36 for locking engagement of bumper 50 with vertebral rod 30.

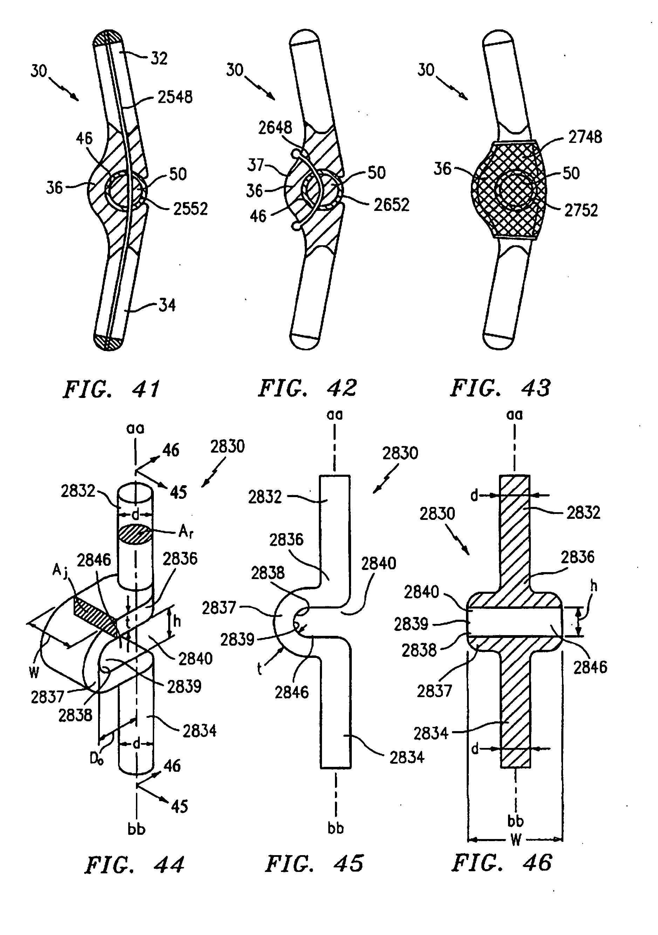

Alternatively, as shown in FIG. 41, the vertebral rod system includes a tether 2548 connected adjacent end portions of sections 32, 34 and extending across a lateral open portion of cavity 46. Bumper 50 includes an exterior surface 2552 configured for engagement with tether 2548 such that bumper 50 is fixed with vertebral rod 30. Alternatively, as shown in FIG. 42, intermediate section 36 includes a first locking part, a tether 2648 connected adjacent upper and lower portions of joint member 37 and extending across a lateral open portion of cavity 46. Bumper 50 includes an exterior surface 2652 configured for engagement with tether 2648 such that bumper 50 is fixed with vertebral rod 30. Alternatively, as shown in FIG. 43, intermediate section 36 includes a first locking part, a mesh 2748 disposed thereabout for capture of bumper 50. Bumper 50 includes an exterior surface 2752 configured for engagement with mesh 2748 such that bumper 50 is fixed with vertebral rod 30.

Referring to FIGS. 44-46, in another alternate embodiment similar to vertebral rods 30, 130 described above, a vertebral rod 2830 includes an upper section 2832 that defines a longitudinal axis *aa* and a lower section 2834 that defines a longitudinal axis *bb.* It is contemplated that, in a first orientation, longitudinal axis *aa* may be disposed at various angular orientations relative to longitudinal axis *bb,* such as, for example, those discussed herein. It is further contemplated that sections 2832, 2834 may include a laterally offset orientation, arcuate portion(s) and alternate lengths, such as, for example, those discussed herein. Movement of vertebral rod 2830 between one or a plurality of orientations is envisioned and may include a range of increasing and decreasing levels of resistance.

An intermediate section 2836 is connected with sections 2832, 2834 and disposed therebetween as a joining section of the components of vertebral rod 2830, similar to the intermediate sections discussed herein. Intermediate section 2836 includes a flexible joint member 2837, which has a U-shaped configuration and defines a corresponding shaped arcuate inner surface 2838 and an open end 2840. Inner surface 2838 has a mid-region 2839, which defines an innermost surface and/or depth of flexible joint member 2837. Mid-region 2839 defines a depth of flexible joint member 2837 as an offset distance Dₒ measured from longitudinal axes *aa* and/or *bb* adjacent open end 2840, to mid-region 2839. It is envisioned that offset distance Dₒ may be in a range of 2-20 millimeters (mm), preferably in a range of 2-15 mm, and most preferably in a range of 2-10 mm.

Open end 2840 defines a spaced apart dimension, such as, for example, a height *h* of the gap or opening defined thereby. Height *h* defines the spaced apart region of intermediate section 2836 disposed between sections 2832, 2834. It is envisioned that height *h* of open end 2840 may be in a range of 3-20 mm, preferably in a range of 3-15 mm, and most preferably in a range of 3-10 mm.

Sections 2832, 2834 each define a dimension of thickness, such as, for example, a diameter d and a corresponding cross-sectional area *Aᵣ*. It is envisioned that diameter d of sections 2832, 2834 may be in a range of 3-11 mm, preferably in a range of 3-9 mm, and most preferably in a range of 3-7 mm. It is further envisioned that cross-sectional area *Aᵣ* can be uniform, non-uniform, consistent or variable. It is contemplated that sections 2832, 2834 may have alternate geometric cross-section configurations, for example, elliptical, rectangular, polygonal, irregular, uniform and non-uniform and have a corresponding cross-sectional area *Aᵣ* based on the particular geometry.

Flexible joint member 2837 is enlarged relative to sections 2832, 2834, as shown in FIG. 46, and defines a width w. It is envisioned that width w of flexible joint member 2837 may be in a range of 3-20 mm, preferably in a range of 3-15 mm, and most preferably in a range of 3-10 mm. Flexible joint member 2837 further defines a thickness *t* and a corresponding cross-sectional area *Aⱼ.* It is envisioned that thickness *t* of flexible joint member 2837 may be in a range of 1-10 mm, preferably in a range of 2-6 mm, and most preferably in a range of 2-4 mm. It is further envisioned that cross-sectional area *Aⱼ* can be uniform, non-uniform, consistent or variable. It is contemplated that flexible joint member 2837 may have alternate geometric cross-section configurations, for example, round, oval, rectangular, polygonal, irregular, uniform and non-uniform and have a corresponding cross-sectional area *Aⱼ* based on the particular geometry.

In one embodiment, thickness *t* of flexible joint member 2837 is less than or equal to diameter *d* of sections 2832, 2834 to provide greater flexibility to vertebral rod 2830. In another embodiment, thickness *t* of flexible joint member 2837 is less than or equal to width *w* of flexible joint member 2837 to provide greater flexibility to vertebral rod 2830. In another embodiment, cross-sectional area *Aⱼ* of flexible joint member 2837 is greater than or equal to 10% of cross-sectional area *Aᵣ* of sections 2832, 2834 to provide greater flexibility to vertebral rod 2830. In another embodiment, width w of flexible joint member 2837 is greater than or equal to diameter *d* of sections 2832, 2834 to provide greater flexibility to vertebral rod 2830. In another embodiment, offset distance Dₒ is greater than or equal to 50% of diameter d of sections 2832, 2834 to provide greater flexibility to vertebral rod 2830. In another embodiment, height *h* of open end 2840 is greater than or equal to 25% of diameter *d* of sections 2832, 2834 to provide greater flexibility to vertebral rod 2830.

Inner surface 2838 defines a cavity 2846 configured for disposal of a resistance member (not shown), such as, for example, those discussed herein. Intermediate section 2836 and the resistance member may include locking parts, similar to those described herein, for locking these components in place. Vertebral rod 2830 may be employed with a surgical procedure for treating a spinal disorder, similar to that discussed above.

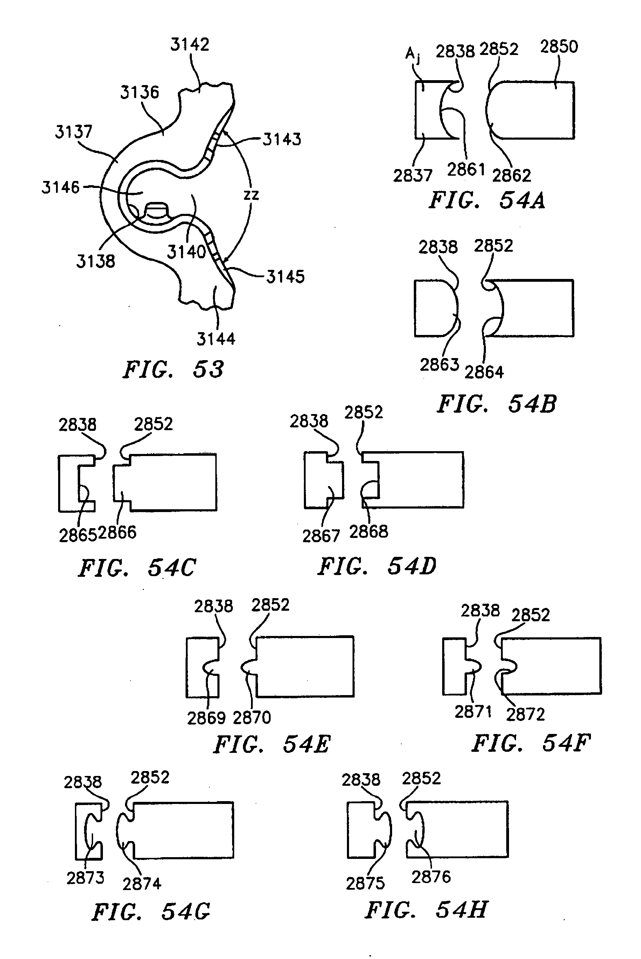

Alternatively, as shown in the alternative embodiments of vertebral rod 2830 illustrated in FIGS. 54A-H, inner surface 2838 defines a first locking part configured for engagement with a second locking part defined by an exterior surface 2852 of a resistance member 2850, similar to the locking part and resistance member components of the vertebral rod embodiments described herein, to lock intermediate section 2836 (FIG. 44) with resistance member 2850. The first locking part of inner surface 2838, as defined by cross-sectional area *Aⱼ* (FIG. 44) of flexible joint member 2837, mates with a correspondingly configured second locking part of exterior surface 2852 for locking engagement. It is envisioned that the locking parts may be defined about substantially all, only a portion or in a specific location of inner surface 2838 and/or exterior surface 2852, respectively. In one embodiment, the first locking part is disposed in the mid portion of flexible joint member 2837 facing open end 2840 (FIG. 44).

For example, as shown in FIG. 54A, inner surface 2838 defines a first locking part, such as a concave surface 2861, which forms a recess or cavity in flexible joint member 2837, as shown by cross-sectional area *Aⱼ.* Concave surface 2861 receives a second locking part, such as convex surface 2862 defined within exterior surface 2852. Convex surface 2862 has an arcuate surface and projects from resistance member 2850 for an interlocking, mating engagement to lock intermediate section 2836 with resistance member 2850. Alternatively, as shown in FIG. 54B, inner surface 2838 defines a convex surface 2863 projecting therefrom, which is received by a concave surface 2864 of exterior surface 2852, for an interlocking, mating engagement, similar to that discussed above.

In another example, as shown in FIG. 54C, inner surface 2838 defines a recess 2865, which is received by a projection 2866 of exterior surface 2852, for an interlocking, mating engagement. Alternatively, as shown in FIG. 54D, inner surface 2838 defines a projection 2867, which is received by a recess 2868 of exterior surface 2852, for an interlocking, mating engagement. In another example, as shown in FIG. 54E, inner surface 2838 defines a longitudinal groove 2869, which is received by a rib 2870 of exterior surface 2852, for an interlocking, mating engagement. Alternatively, as shown in FIG. 54F, inner surface 2838 defines a rib 2871, which is received by a longitudinal groove 2872 of exterior surface 2852, for an interlocking, mating engagement. In another example, as shown in FIG. 54G, inner surface 2838 defines a channel 2873, which receives a dove-tail projection 2874 of exterior surface 2852, for an interlocking, mating engagement. Alternatively, as shown in FIG. 54H, inner surface 2838 defines a dove-tail projection 2875, which is received by a channel 2876 of exterior surface 2852, for an interlocking, mating engagement. It is contemplated that vertebral rods 30, 130 and 930 described herein may similarly include the locking parts described with regard to FIGS. 54A-H.

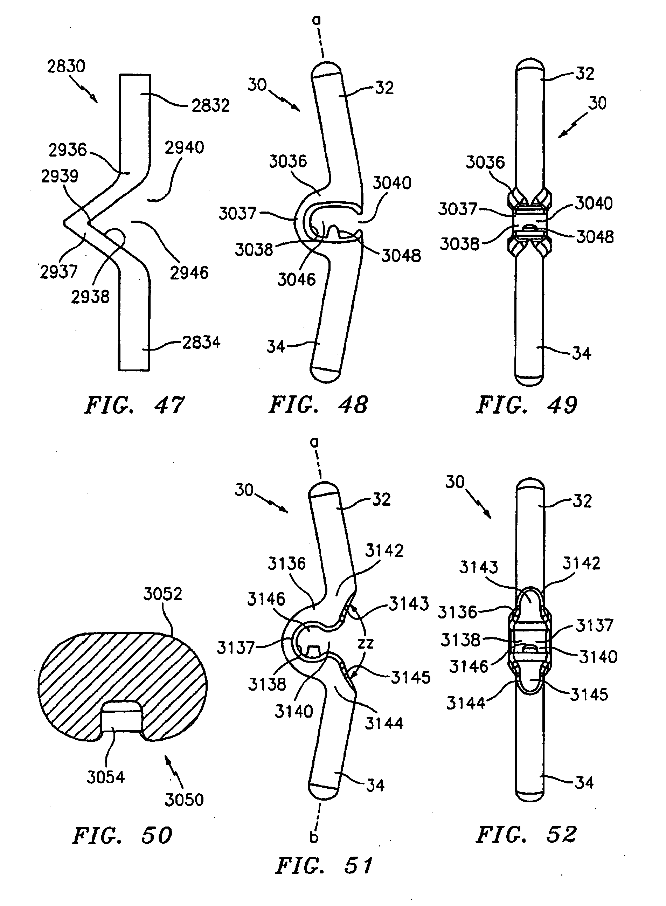

Referring to FIG. 47, in an alternate embodiment of vertebral rod 2830 similar to that described above with regard to FIGS. 44-46, an intermediate section 2936 is connected with sections 2832, 2834 and disposed therebetween as a joining section of the components of vertebral rod 2830. Intermediate section 2936 includes a flexible joint member 2937, which has a V-shaped configuration and defines a correspondingly shaped angled inner surface 2938 and an open end 2940. Inner surface 2938 has a mid-line 2939, which defines an innermost surface and/or depth of flexible joint member 2937. Inner surface 2938 defines an angled cavity 2946 configured for disposal of a resistance member, such as, for example, those discussed herein.

Referring to FIGS. 48-50, in an alternate embodiment of vertebral rod 30, similar to that described with regard to FIGS. 1-3, an intermediate section 3036 is connected with sections 32, 34 and disposed therebetween as a joining section of the components of vertebral rod 30. Intermediate section 3036 includes a flexible joint member 3037, which has an elliptically shaped configuration and defines inner surface 3038 and an open end 3040.

Inner surface 3038 defines an elliptically shaped cavity 3046 and a post 3048, similar to post 48 described above. Cavity 3046 is configured for disposal of a resistance member, such as, for example, an oblong shaped bumper 3050, as shown in FIG. 50 and similar to bumper 50 described above. Bumper 3050 has an exterior surface 3052 that defines an opening 3054. Opening 3054 receives post 3048 for fixed mounting of bumper 3050 with vertebral rod 30 to lock these components of the vertebral rod system in place.

Referring to FIGS. 51-53, in an alternate embodiment of vertebral rod 30, similar to that described with regard to FIGS. 1-3, an intermediate section 3136 is connected with sections 32, 34 and disposed therebetween as a joining section of the components of vertebral rod 30. Intermediate section 3136 include a flexible joint member 3137 having a posterior offset configuration, which is C-shaped and defines inner surface 3138 and an open end 3140.

Upper section 32 is disposed adjacent to open end 3140 such that section 32 and intermediate section 3136 define an upper transition 3142. Upper transition 3142 defines a front face 3143. Lower section 34 is disposed adjacent to open end 3140 such that section 34 and intermediate section 3136 define an lower transition 3144. Lower Transition 3144 defines a front face 3145. Front face 3143 is disposed at an angle zz relative to front face 3145. Angle zz is desirably in a range of 60-179 degrees, and most desirably in a range of 90-160 degrees.

Section 32 extends from upper transition 3142 and section 34 extends from lower transition 3144 such that longitudinal axis *a* of section 32 is disposed in a non-parallel relation with longitudinal axis *b* of section 34. Intermediate section 3136 extends from transitions 3142, 3144 in a posterior offset configuration. The posterior offset configuration has a larger moment as defined by the distance between the central axis of vertebral rod 30 and flexible joint member 3137. This configuration increases flexibility of vertebral rod 30, facilitating bending thereof.

Inner surface 3138 defines a cavity 3146 configured for disposal of a resistance member (not shown), such as, for example, those described above, to lock these components of the vertebral rod system in place.

It will be understood that various modifications may be made to the embodiments disclosed herein. Therefore, the above description should not be construed as limiting, but merely as exemplification of the various embodiments. Those skilled in the art will envision other modifications within the scope and spirit of the claims appended hereto.

## Claims

1. A vertebral rod (30) comprising: a first elongated section (32) extending along a longitudinal axis (a) and defining a first thickness; a second elongated section (32) extending along a longitudinal axis (b) and defining a second thickness; an intermediate section (36) disposed between the first section (32) and the second section (34) and defining a third thickness, the third thickness having a dimension being less than a dimension of at least one of the first thickness and the second thickness, the intermediate section including a flexible joint member (37) which has a C-shaped configuration and defines a correspondingly shaped arcuate inner surface (38) that defines a cavity (46) and an open end; and a resistance member (50) disposed within the cavity (46) and having an exterior surface engaging the inner surface (38) in a close fitting engagement, and wherein in a first orientation of the vertebral rod (30), in which no flexion or extension force is applied to vertebral rod (30), longitudinal axis (a) is disposed at an angle (X) to longitudinal axis (b) about joint member (37), **characterized in that** the angle (X) is in the range of 135° to less than 180°.

2. A vertebral rod (30) according to claim 1, wherein the intermediate section (36) defines a width relative to each of the first thickness and the second thickness such that the dimension of the third thickness is less than or equal to a dimension of the width.

3. A vertebral rod (30) according to claim 2, wherein the dimension of the width is greater than or equal to at least one of the dimension of the first thickness and the second thickness.

4. A vertebral rod (30) according to claim 1, wherein the third thickness is in a range of 2-6mm.

5. A vertebral rod (30) according to claim 3, wherein the width is in a range of 3-10mm.

6. A vertebral rod (30) according to claim 3, wherein the first and second sections (32, 34) are fabricated from a first material and the intermediate section (36) is fabricated from a second material.

## Patentansprüche

1. Wirbelstange (30), aufweisend: einen ersten langgestreckten Abschnitt (32), der sich entlang einer Längsachse (a) erstreckt und eine erste Dicke definiert, einen zweiten langgestreckten Abschnitt (32), der sich entlang einer Längsachse (b) erstreckt und eine zweite Dicke definiert, einen Zwischenabschnitt (36), der zwischen dem ersten Abschnitt (32) und dem zweiten Abschnitt (34) angeordnet ist und der eine dritte Dicke definiert, wobei die dritte Dicke eine Abmessung hat, die kleiner ist als eine Abmessung von wenigstens einer von der ersten Dicke und der zweiten Dicke, wobei der Zwischenabschnitt ein flexibles Verbindungselement (37) aufweist, welches eine C-förmige Konfiguration hat und eine korrespondierend geformte gekrümmte innere Fläche (38) definiert, welche eine Kavität (46) und ein offenes Ende definiert, und ein Widerstandselement (50), welches in der Kavität (46) angeordnet ist und welches eine äußere Fläche hat, die mit der inneren Fläche (38) im Eingriff ist in einem Enge-Einpassung-Eingriff, und wobei in einer ersten Orientierung der Wirbelstange (30), in welcher keine Beugungs- oder Extensions-Kraft auf die Wirbelstange (30) ausgeübt ist, die Längsachse (a) zur Längsachse (b) um das Verbindungselement (37) in einem Winkel (X) angeordnet ist, **dadurch gekennzeichnet, dass** der Winkel (X) in einem Bereich von 135° bis kleiner als 180° ist.

2. Wirbelstange (30) gemäß Anspruch 1, wobei der Zwischenabschnitt (36) eine Weite relativ zu jeder der ersten Dicke und der zweiten Dicke definiert, sodass die Abmessung der dritten Dicke kleiner als oder gleich der Abmessung der Weite ist.

3. Wirbelstange (30) gemäß Anspruch 2, wobei die Abmessung der Weite größer als oder gleich wenigstens einer von der Abmessung der ersten Dicke und der zweiten Dicke ist.

4. Wirbelstange (30) gemäß Anspruch 1, wobei die dritte Dicke in einem Bereich von 2-6mm ist.

5. Wirbelstange (30) gemäß Anspruch 3, wobei die Weite in einem Bereich von 3-10mm ist.

6. Wirbelstange (30) gemäß Anspruch 3, wobei der erste und der zweite Abschnitt (32, 34) aus einem ersten Material hergestellt sind und der Zwischenabschnitt (36) aus einem zweiten Material hergestellt ist.

## Revendications

1. Une tige vertébrale (30), comportant: une première section allongée (32) s'étendant le long d'un axe longitudinal (a) et définissant une première épaisseur ; une deuxième section allongée (32) s'étendant le long d'un axe longitudinal (b) et définissant une deuxième épaisseur ; une section intermédiaire (36) disposée entre la première section (32) et la deuxième section (34) et définissant une troisième épaisseur, la troisième épaisseur ayant une dimension qui est inférieure à une dimension d'au moins une de la première épaisseur et la deuxième épaisseur, la section intermédiaire incluant un élément de joint flexible (37) qui a une configuration en forme de C et définit une surface intérieure (38) arquée de forme correspondante définissant une cavité (46) et une extrémité ouverte ; et un élément de résistance (50) disposé dans la cavité (46) et ayant une surface extérieure en prise de la surface intérieure (38) dans un engagement par fixation étroite, et dans laquelle, dans une première orientation de la tige vertébrale (30) dans laquelle aucune force de flexion ou d'extension n'est appliquée à la tige vertébrale (30), l'axe longitudinal (a) est disposé dans un angle (X) par rapport à l'axe longitudinal (b) autour de l'élément de joint (37), **caractérisée en ce que** l'angle (X) est dans une plage de 135° à moins de 180°.

2. Une tige vertébrale (30) selon la revendication 1, dans laquelle la section intermédiaire (36) définit une largeur par rapport à chacune de la première épaisseur et la deuxième épaisseur, de sorte que la dimension de la troisième épaisseur est inférieure ou égale à une dimension de la largeur.

3. Une tige vertébrale (30) selon la revendication 2, dans laquelle la dimension de la largeur est supérieure ou égale à au moins une de la dimension de la première épaisseur et la deuxième épaisseur.

4. Une tige vertébrale (30) selon la revendication 1, dans laquelle la troisième épaisseur est dans une plage de 2 mm à 6 mm.

5. Une tige vertébrale (30) selon la revendication 3, dans laquelle la largeur est dans une plage de 3 mm à 10 mm.

6. Une tige vertébrale (30) selon la revendication 3, dans laquelle les première et deuxième sections (32, 34) sont fabriquées d'un premier matériau et la section intermédiaire (36) est fabriquée d'un deuxième matériau.
